# EUROPEAN PATENT SPECIFICATION

(11) **EP 2 694 039 B1**
(45) Date of publication and mention of the grant of the patent: **21.09.2016**
(21) Application number: 12717499.3
(22) Date of filing: 29.03.2012
(51) Int. Cl.: A61K 47/02, A61K 31/4188, A61K 9/20

(54) **SOLID PREPARATION**
FESTE ZUBEREITUNG
PRÉPARATION SOLIDE

(30) Priority: 01.04.2011 JP 2011082301
(43) Date of publication of application: 12.02.2014
(73) Proprietor: Takeda Pharmaceutical Company Limited, Osaka-shi, Osaka 541-0045 (JP)
(72) Inventor: HOSHINA, Wataru, Osaka-shi Osaka 532-0024 (JP); MISAKI, Masafumi, Osaka-shi Osaka 532-0024 (JP)
(74) Representative: Kingsbury, Oliver William
(86) International application number: PCT/JP2012/059276
(87) International publication number: WO 2012/133918

(56) References cited:
- US-A1- 2009 053 308

## Description

### Technical Field of the Invention

The present invention relates to a solid preparation containing 6-((7S)-7-hydroxy-6,7-dihydro-5H-pyrrblo[1,2-c]imidazol-7-yl)-N-methyl-2-naphthamide or a salt thereof, and a method of stabilizing 6-((7S)-7-hydroxy-6,7-dihydro-5H-pyrrolo[1,2-c]imidazol-7-yl)-N-methyl-2-naphthamide or a salt thereof in the solid preparation.

### Background of the Invention

6-((7S)-7-Hydroxy-6,7-dihydro-5H-pyrrolo[1,2-c]imidazol-7-yl)-N-methyl-2-naphthamide is known to be a compound useful for the prophylaxis or treatment of tumors such as prostate cancer, breast cancer and the like (patent document 1). As a preparation containing 6-((7S)-7-hydroxy-6,7-dihydro-5H-pyrrolo[1,2-c]imidazol-7-yl)-N-methyl-2-naphthamide, preparations described in patent documents 2 to 4 are known. [Document List]

### [Patent Documents]

patent document 1: WO2002/040484
patent document 2: WO2004/075890
patent document 3: WO2004/082679
patent document 4: WO2006/093353

### [Summary of the Invention]

### Problems to be Solved by the Invention

The present inventors have found a new problem of achieving a high content of the active ingredient and a downsized preparation for the improvement of administration compliance of a solid preparation containing 6-((7S)-7-hydroxy-6,7-dihydro-5H-pyrrolo[1,2-c]imidazol-7-yl)-N-methyl-2-naphthamide or a salt thereof as an active ingredient.

In solving the problem of achieving a high content of the active ingredient and a downsized preparation, moreover, new problems of suppression of varying weights of preparation and varying contents of the active ingredient, as well as improvement of preparation preservation stability (suppression of production or increase of dehydrated form and related substances, which are decomposed products of the active ingredient) were found.

Since variation in the weights of preparation and the contents of the active ingredient may lead to severe side effects in patients, it is desirable to reduce such variations as much as possible. As for the preparation preservation stability, it is desirable to suppress production or increase of dehydrated form and related substances, which are decomposed products of the active ingredient, as much as possible, so that the efficacy of the active ingredient can be appropriately exerted in patients.

The present invention provides a solid preparation containing 6-((7S)-7-hydroxy-6,7-dihydro-5H-pyrrolo[1,2-c]imidazol-7-yl)-N-methyl-2-naphthamide or a salt thereof at a high content (e.g., 40 wt% or more) as an active ingredient, wherein variation in the weight and the content is suppressed.

The present invention also aims to provide a solid preparation wherein the active ingredient is stabilized, and a stabilizing method thereof. Here, the stabilization of the active ingredient means that the production or increase of dehydrated form and related substances, which are decomposed products of the active ingredient contained in the solid preparation, is suppressed.

### Means of Solving the Problems

The present inventors have conducted intensive studies in an attempt to solve the aforementioned problems and found that a solid preparation containing 6-((7S)-7-hydroxy-6,7-dihydro-5H-pyrrolo[1,2-c]imidazol-7-yl)-N-methyl-2-naphthamide or a salt thereof as an active ingredient, D-mannitol and an alkaline earth metal salt selected from, magnesium aluminometasilicate and calcium silicate provides superior effects of high content (e.g., 40 wt% or more) of the active ingredient, suppression of variation in preparation weight and active ingredient content, and improved preparation preservation stability (suppression of production or increase of dehydrated form and related substances, which are decomposed products of the active ingredient). The present inventors have completed the present invention based on these findings.

Accordingly, the present invention is as follows.
[1] A solid preparation comprising
   (1) 50-80 wt% of 6-((7S)-7-hydroxy-6,7-dihydro-5H-pyrrolo[1,2-c]imidazol-7-yl)-N-methyl-2-naphthamide or a salt thereof,
   (2) D-mannitol and
   (3) an alkaline earth metal salt selected from magnesium aluminometasilicate and calcium silicate
   (hereinafter sometimes to be abbreviated as the solid preparation of the present invention).
[2] The solid preparation of the above-mentioned [1], wherein the alkaline earth metal salt is magnesium
   aluminometasilicate.
[3] The solid preparation of the above-mentioned [1], wherein the alkaline earth metal salt is basic magnesium aluminometasilicate.
[4] The solid preparation of the above-mentioned [1], wherein the D-mannitol is produced by a spray dry production method.
[5] The solid preparation of the above-mentioned [1], further comprising hydroxypropylcellulose.
[6] A method of stabilizing 6-((7S)-7-hydroxy-6,7-dihydro-5H-pyrrolo[1,2-c]imidazol-7-yl)-N-methyl-2-naphthamide or a salt thereof, comprising adding (1) an alkaline earth metal salt selected from magnesium aluminometasilicate and calcium silicate and (2) D-mannitol to a solid preparation containing 6-((7S)-7-hydroxy-6,7-dihydro-5H-pyrrolo[1,2-c]imidazol-7-yl)-N-methyl-2-naphthamide or a salt thereof (hereinafter sometimes to be abbreviated as the stabilization method of the present invention).
[7] The method of the above-mentioned [6], wherein the alkaline earth metal salt is basic magnesium
   aluminometasilicate.

### Effect of the Invention

According to the present invention, a solid preparation containing 6-((7S)-7-hydroxy-6,7-dihydro-5H-pyrrolo[1,2-c]imidazol-7-yl)-N-methyl-2-naphthamide or a salt thereof at a high content (e.g., 40 wt% or more) as an active ingredient, which can suppress variation in preparation weight and content of active ingredient can be provided. According to the present invention, moreover, a solid preparation wherein the active ingredient is stabilized, namely, a solid preparation wherein preparation preservation stability is improved, and production or increase of dehydrated form and related substances, which are decomposed products of the active ingredient, is suppressed, can be provided.

### [Description of Embodiments]

6-((7S)-7-Hydroxy-6,7-dihydro-5H-pyrrolo[1,2-c]imidazol-7-yl)-N-methyl-2-naphthamide (to be also referred to as compound A in the present specification) or a salt thereof can be produced by a known method, for example, the method described in WO2002/040484 or a method analogous thereto.

Examples of the salt of compound A include acid addition salts, for example, inorganic acid salts (e.g., hydrochloride, sulfate, hydrobromide, phosphate), and organic acid salts (e.g., acetate, trifluoroacetate, succinate, maleate, fumarate, propionate, citrate, tartrate, lactate, oxalate, methanesulfonate, p-toluenesulfonate). The salt of compound A may be a hydrate. Of compound A and a salt thereof, preferred is compound A.

The content of compound A or a salt thereof in the solid preparation of the present invention is generally 50 - 80 wt%, preferably 60 - 70 wt%.

The solid preparation of the present invention contains D-mannitol.

To provide a downsized preparation, D-mannitol to be used in the present invention preferably has an average particle size of 50 µm - 250 µm, more preferably 100 µm - 200 µm.

Examples of D-mannitol to be used in the present invention include D-mannitol produced by the spray dry production method (e.g., PEARLITOL 200SD (trade name) (manufactured by ROQUETTE), PEARLITOL 100SD (trade name) (manufactured by ROQUETTE), PARTECK 100M (trade name) (manufactured by Merck), and PARTECK 200M (trade name) (manufactured by Merck)). Of these, PEARLITOL 200SD (trade name) (manufactured by ROQUETTE) and PEARLITOL 100SD (trade name) (manufactured by ROQUETTE) are preferable, and PEARLITOL 200SD (trade name) (manufactured by ROQUETTE) is more preferable, from the aspects of manufacturability.

The content of D-mannitol in the solid preparation of the present invention is generally 5 - 45 wt%, preferably 10 - 30 wt%, more preferably 15 - 25 wt%.

The solid preparation of the present invention contains an alkaline earth metal salt selected from magnesium aluminometasilicate and calcium silicate. The solid preparation of the present invention may contain both magnesium aluminometasilicate and calcium silicate.

The alkaline earth metal salt selected from magnesium aluminometasilicate and calcium silicate to be used in the present invention is preferably magnesium aluminometasilicate, more preferably basic magnesium aluminometasilicate, from the aspects of improved preservation stability of the solid preparation (stabilization of compound A or a salt thereof) and suppression of variation in the weight of the solid preparation and the content of the active ingredient.

In addition, as the alkaline earth metal salt selected from magnesium aluminometasilicate and calcium silicate to be used in the present invention is preferably basic magnesium aluminometasilicate or basic calcium silicate, more preferably basic magnesium aluminometasilicate, from the aspect of stabilization of compound A or a salt thereof.

In the present specification, basic magnesium aluminometasilicate generally shows pH of 8.5 - 10.0 when a sample thereof (2 g) is weighed, water is added to the total amount of 50 mL, the mixture is stirred, left standing for 2 min, and measured by a pH meter.

In the present specification, basic calcium silicate generally shows pH of 8.5 - 9.8 when a sample thereof (5.0 g) is weighed, water is added to the total amount of 100 mL, the mixture is stirred and centrifuged, and the supernatant is measured by a pH meter.

As basic magnesium aluminometasilicate and basic calcium silicate, commercially available products can also be used. Examples of basic magnesium aluminometasilicate include Neusilin FL1 and Neusilin FL2 (trade name) (both manufactured by Fuji Chemical Industry Co., Ltd.). Examples of basic calcium silicate include Florite RE (trade name) (manufactured by Eisai Food & Chemical Co., Ltd.).

The content of the alkaline earth metal salt in the solid preparation of the present invention, which is selected from magnesium aluminometasilicate and calcium silicate, is generally 0.5 - 10 wt%, preferably 0.5 - 5 wt%, more preferably 0.5 - 2.5 wt%.

The solid preparation of the present invention may further contain crystalline cellulose to optimize physicochemical property of the preparation (e.g., manufacturability, tablet disintegration property, tablet hardness).

When the solid preparation of the present invention contains crystalline cellulose, the content of the crystalline cellulose in the solid preparation is generally 1 - 30 wt%, preferably 2 - 15 wt%, more preferably 3 - 10 wt%.

The solid preparation of the present invention preferably further contains hydroxypropylcellulose to optimize physicochemical property of the preparation (e.g., manufacturability, tablet hardness). When the solid preparation of the present invention contains hydroxypropylcellulose, the content of the hydroxypropylcellulose in the solid preparation is generally 1 - 10 wt%, preferably 3 - 5 wt%, more preferably 2 - 4 wt%.

The solid preparation of the present invention preferably further contains low-substituted hydroxypropylcellulose to optimize physicochemical property of the preparation (e.g., dissolution property of active substance, manufacturability, tablet hardness).

As the low-substituted hydroxypropylcellulose to be used in the present invention, for example, low-substituted hydroxypropylcellulose wherein the content of a hydroxypropoxy group is 5 - 16% can be used.

The grade of low-substituted hydroxypropylcellulose to be used in the present invention is, for example, LH-11, LH-21, LH-22 or LH-B1 (trade name) (manufactured by Shin-Etsu Chemical Co., Ltd.).

When the solid preparation of the present invention contains low-substituted hydroxypropylcellulose, the content of the low-substituted hydroxypropylcellulose in the solid preparation is generally 2 - 20 wt%, preferably 5 - 15 wt%, more preferably 7 - 13 wt%.

The solid preparation of the present invention preferably further contains a surfactant to optimize physicochemical property of the preparation (e.g., manufacturability, tablet disintegration property, dissolution property).

Examples of the surfactant to be used in the present invention include sodium lauryl sulfate, sucrose ester of fatty acid, polysorbate 20, polysorbate 60, polysorbate 80, and polyoxyethylene hydrogenated castor oil 60. The surfactant to be used in the present invention is preferably polysorbate 80 from the aspect of the manufacturability of the solid preparation of the present invention.

When the solid preparation of the present invention contains a surfactant, the content of the surfactant in the solid preparation is generally 0.05 - 5 wt%, preferably 0.1 - 3 wt%, more preferably 0.3 - 1.5 wt%.

The solid preparation of the present invention can contain a pharmaceutically acceptable carrier besides the above-mentioned components, as long as it does not inhibit the effect of the present invention. As the pharmaceutically acceptable carrier, various organic or inorganic carrier substances conventionally used as preparation materials can be used. They are appropriately added as, for example, excipient, binder, disintegrant, glidant or lubricant in an appropriate amount.

Examples of the excipient include sugar alcohol other than D-mannitol (e.g., D-sorbitol, erythritol, xylitol), lactose, sucrose, glucose, malt sugar, corn starch, wheat starch, light anhydrous silicic acid, dextrin, carboxymethyl starch, gelatin, magnesium oxide, calcium phosphate, calcium carbonate, and calcium sulfate.

Examples of the binder include gelatin, pullulan, hydroxypropyl methylcellulose (HPMC), methylcellulose (MC), polyvinylpyrrolidone (PVP), macrogol, gum arabic, dextran, polyvinyl alcohol (PVA), and starch glue.

Examples of the disintegrant include carmellose, carmellose calcium, crosslinked polyvinylpyrrolidone, carmellose sodium, croscarmellose sodium, sodium starch glycolate, crospovidone, cation exchange resin, partly pregelatinized starch, and corn starch.

Examples of the glidant include light anhydrous silicic acid, and hydrated silicon dioxide.

Examples of the lubricant include stearic acid, magnesium stearate, calcium stearate, talc, waxes, DL-leucine, sodium lauryl sulfate, magnesium lauryl sulfate, macrogol, and light anhydrous silicic acid.

The solid preparation of the present invention may be coated with a coating agent, a film coating agent and the like according to a method known per se, for the purpose of masking the taste of compound A or a salt thereof, improvement of light stability, improvement of appearance, controlled release and the like.

As the coating agent, polymers such as hydroxypropyl methylcellulose (for example, hydroxypropyl methylcellulose 2910), ethylcellulose, hydroxypropylcellulose and the like are used. As the film coating agent, polymers such as hydroxypropylcellulose (HPC), hydroxypropyl methylcellulose (HPMC), polyvinylpyrrolidone (PVP), ethylcellulose, polyvinyl acetal diethylamino acetate, cellulose acetate phthalate, methacrylic acid copolymers (e.g., methyl methacrylate-methacrylic acid copolymer (Eudragit (trade name) L100, S100, manufactured by Rohm), methacrylic acid-ethyl acrylate copolymer (Eudragit L100-55, L30D-55), methacrylic acid-methyl acrylate-methyl methacrylate copolymer (Eudragit FS30D (trade name), manufactured by Rohm)), hydroxypropyl methylcellulose phthalate (HP-55 (trade name), HP-50 (trade name), manufactured by Shin-Etsu Chemical Co., Ltd.), carboxymethyl ethylcellulose (CMEC, manufactured by Freund Corporation), hydroxypropylcellulose acetate succinate (HPMCAS manufactured by Shin-Etsu Chemical Co., Ltd.), polyvinyl acetate phthalate, shellac and the like are used. These may be used alone or two or more kinds of polymers may be used in combination, or two or more kinds of polymers may be applied successively.

The above-mentioned coating agent and film coating agent may contain polyethylene glycol (for example, polyethylene glycol 6000 (macrogol 6000), polyethylene glycol 8000), Tween 80, titanium oxide, ferric oxide (e.g., red ferric oxide, yellow ferric oxide) and the like. Specific preferable examples of the film coating agent include Opadry Red (trade name) (manufactured by Colorcon), and Opadry Yellow (trade name) (manufactured by Colorcon).

Specific preferable examples of the solid preparation of the present invention include the following.
(1) A solid preparation comprising compound A, D-mannitol, magnesium aluminometasilicate and hydroxypropylcellulose.
(2) The solid preparation of the above-mentioned (1), further comprising crystalline cellulose, sodium starch glycolate and magnesium stearate.
(3) The solid preparation of the above-mentioned (1), further comprising low-substituted hydroxypropylcellulose, polysorbate 80 and magnesium stearate.
(4) The solid preparation of the above-mentioned (2) or (3), further comprising hydroxypropyl methylcellulose, polyethylene glycol, titanium oxide and a colorant (red ferric oxide and/or yellow ferric oxide).

Examples of the dosage form of the solid preparation of the present invention include tablet (e.g., core tablet, film-coated tablet) and the like.

The solid preparation of the present invention can be produced by a method conventionally used in the pharmaceutical field.

For example, compound A or a salt thereof, D-mannitol, an alkaline earth metal salt selected from magnesium aluminometasilicate and calcium silicate, and an optional carrier or additive (e.g., excipient such as crystalline cellulose and the like, low-substituted hydroxypropylcellulose) are mixed, the mixture is granulated using an aqueous solution of a binder (e.g., hydroxypropylcellulose) containing an optional carrier or additive (e.g., surfactant such as polysorbate 80 and the like), and the granules are sieved when desired. To the obtained sieved powder are added an optional carrier or additive (e.g., disintegrant such as sodium starch glycolate, hydroxypropylcellulose and the like, lubricant such as magnesium stearate and the like), they are mixed, molded and further dried when desired, whereby the solid preparation of the present invention is produced. Mixing and granulation can be performed using, for example, a fluid bed dryer granulator and the like. Molding can be performed by tableting using, for example, a single punch tableting machine.

A film-coated tablet can be produced by, for example, coating a core tablet obtained by the above-mentioned method, by spraying an aqueous solution of a film coating agent (e.g., a mixture of film coating base such as hydroxypropyl methylcellulose 2910 and the like, plasticizer such as polyethylene glycol 6000 and the like, and dye such as titanium oxide, red ferric oxide, yellow ferric oxide and the like) by a film coating machine and the like.

The solid preparation of the present invention is preferably produced by a fluid bed granulation method. A solid preparation produced by a fluid bed granulation method, particularly a tablet, shows a remarkable effect of the present invention.

The weight of the solid preparation of the present invention is generally 150 - 500 mg, preferably 150 - 350 mg.

The solid preparation of the present invention can be downsized by the use of D-mannitol produced by a spray dry production method. To be precise, when D-mannitol produced by a spray dry production method is used, the weight of a solid preparation containing compound A or a salt thereof at a high content (e.g., 40% or more) can be generally set to not more than 500 mg, preferably not more than 400 mg, more preferably not more than 200 mg.

The solid preparation of the present invention has superior effects as a medicament, and particularly shows a superior inhibitory activity against steroid C_{17,20} lyase. Since the solid preparation of the present invention is low in toxicity and has fewer side effects, it is useful for mammals (e.g., human, bovine, horse, swine, dog, cat, monkey, mouse, rat, particularly human) as, for example, (i) an androgen or estrogen reducer, (ii) an agent for the prophylaxis or treatment of various androgen- or estrogen-related diseases, such as (1) primary cancer, metastasis or recurrence of malignant tumor (e.g., prostate cancer, breast cancer, uterine cancer, ovarian cancer etc.), (2) various symptoms associated with those cancers (e.g., pain, cachexia etc.), (3) prostatic hypertrophy, virilism, hirsutism, male pattern alopecia, precocious puberty, endometriosis, uterus myoma, adenomyosis of uterus, mastopathy, polycystic ovary syndrome and the like, or (iii) an agent for the treatment or prophylaxis of androgen-independent cancer (e.g., androgen-independent prostate cancer).

In the present specification, an androgen- or estrogen-reducer means a medicament having an action to suppress androgen production and subsequent estrogen production (estrogen is synthesized with androgen as a substrate).

The solid preparation of the present invention can be administered orally and safely to a mammal.

While the dose of the solid preparation of the present invention varies depending on the subject of administration, administration frequency and the like, the preparation shows effectiveness over a wide range. For example, the daily dose of the solid preparation of the present invention to an adult patient with solid tumor (e.g., prostate cancer patient) is generally about 100 to about 1200 mg, preferably about 300 to about 1000 mg, more preferably about 400 to about 800 mg, as an effective amount of compound A or a salt thereof contained in the solid preparation of the present invention. When the solid preparation is combined with other anti-cancer agent, the dose thereof is generally lower than the above doses. However, the dose of the solid preparation to be actually administered is determined according to various preparation forms, age, body weight and sex of the patient, disease level, administration route, term and interval of the administration, and the like, and can be altered at any time based on the judgment of the doctor.

The term and interval of the administration of the solid preparation of the present invention vary depending on various conditions, and can be altered at any time based on the judgment of the doctor. Divided administration, consecutive administration, intermittent administration, high dose short period administration, repeat administration and the like can be employed. For oral administration, for example, the daily dose is desirably administered in one to several portions a day (especially two or three doses per day). In addition, the solid preparation of the present invention can also be administered as a sustained-release preparation.

The present invention also relates to a method of stabilizing compound A or a salt thereof, comprising adding an alkaline earth metal salt selected from magnesium aluminometasilicate and calcium silicate to a solid preparation containing compound A or a salt thereof. In the stabilization method of the present invention, the "solid preparation containing compound A or a salt thereof" may contain both magnesium aluminometasilicate and calcium silicate.

The amount of the alkaline earth metal salt selected from magnesium aluminometasilicate and calcium silicate, which is used for the stabilization method of the present invention is, for example, a range similar to the content of the alkaline earth metal salt selected from magnesium aluminometasilicate and calcium silicate in the above-mentioned solid preparation of the present invention.

As an alkaline earth metal salt selected from magnesium aluminometasilicate and calcium silicate, which is used for the stabilization method of the present invention, magnesium aluminometasilicate (particularly, basic magnesium aluminometasilicate), and basic calcium silicate are preferable, and basic magnesium aluminometasilicate is more preferable.

In the stabilization method of the present invention, the content of compound A or a salt thereof in the "solid preparation containing compound A or a salt thereof" is, for example, a range similar to the content of compound A or a salt thereof in the above-mentioned solid preparation of the present invention. The "solid preparation containing compound A or a salt thereof" in the stabilization method of the present invention may contain components similar to, for example, the components explained for the above-mentioned solid preparation of the present invention, and can be produced in the same manner.

The stabilization method of the present invention shows superior effects in a solid preparation produced by a fluid bed granulation method, particularly a tablet.

The stabilization method of the present invention also includes a method of stabilizing compound A or a salt thereof, comprising adding
(1) an alkaline earth metal salt selected from magnesium aluminometasilicate and calcium silicate and
(2) D-mannitol
to a solid preparation containing compound A or a salt thereof.

In the stabilization method of the present invention, the "solid preparation containing compound A or a salt thereof" may contain magnesium aluminometasilicate, calcium silicate and D-mannitol.

The amount of the D-mannitol to be used in the stabilization method of the present invention is, for example, a range similar to the content of D-mannitol in the above-mentioned solid preparation of the present invention. Examples

The present invention is explained in more detail in the following by referring to Comparative Examples, Reference Examples, Examples and Experimental Examples, which are not to be construed as limitative.

In the following Comparative Examples, Reference Examples, Examples and Experimental Examples, D-mannitol (PEARLITOL 200SD (trade name), manufactured by ROQUETTE), crystalline cellulose, hydroxypropylcellulose, sodium starch glycolate, croscarmellose sodium, light anhydrous silicic acid (AEROSIL 200 (trade name), manufactured by NIPPON AEROSIL), magnesium stearate, polysorbate 80 (POLYSORBATE 80 (trade name), manufactured by Sanyo Chemical Industries, Ltd.), low-substituted hydroxypropylcellulose (LH-21 (trade name), manufactured by Shin-Etsu Chemical Co., Ltd.), hydroxypropyl methylcellulose 2910 (TC-5 (trade name), manufactured by Shin-Etsu Chemical Co., Ltd.), macrogol 6000 (MACROGOL 6000 (trade name), manufactured by Sanyo Chemical Industries, Ltd.), titanium oxide (Titanium oxide (trade name), manufactured by Freund Corporation) used are the Japanese Pharmacopoeia Fifteenth Edition compatible products, crospovidone, calcium silicate (Florite RE (trade name), manufactured by Eisai Food & Chemical Co., Ltd.), and red ferric oxide (Red iron oxide (trade name), manufactured by LCW) used are Japanese Pharmaceutical Excipients 2003 compatible products, and magnesium aluminometasilicate (Neusilin FL2 (trade name), manufactured by Fuji Chemical Industry Co., Ltd.) used is the Japanese Pharmacopoeia Japanese Pharmaceutical Codex 2002 compatible product. As a film coating agent, Opadry Red (trade name) (manufactured by Colorcon) which is a premix of hydroxypropyl methylcellulose 2910, macrogol 6000, titanium oxide and red ferric oxide was used and, as a film coating agent, Opadry Yellow (trade name) (manufactured by Colorcon) which is a premix of hydroxypropyl methylcellulose 2910, macrogol 6000, titanium oxide and yellow ferric oxide was used.

### Comparative Example 1

Compound A (267.4 g), D-mannitol (98.4 g) and crystalline cellulose (21.4 g) were placed in a fluid bed dryer granulator (manufactured by POWREX CORPORATION), preheated and mixed, and an aqueous solution (213.9 g) of hydroxypropylcellulose (12.8 g) was sprayed to give a granulated powder. The total amount of the obtained granulated powder was passed through a sieve No. 20 to give a sieved powder. The obtained sieved powder (360 g), sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 1

Compound A (267.4 g), D-mannitol (85.6 g), crystalline cellulose (21.4 g), and magnesium aluminometasilicate (12.8 g) were placed in a fluid bed dryer granulator (manufactured by POWREX CORPORATION), preheated and mixed, and an aqueous solution (213.9 g) of hydroxypropylcellulose (12.8 g) was sprayed to give a granulated powder. The total amount of the obtained granulated powder was passed through a sieve No. 20 to give a sieved powder. The obtained sieved powder (360 g), sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Comparative Example 2

Compound A (267.4 g), D-mannitol (94.1 g), crystalline cellulose (21.4 g), and light anhydrous silicic acid (4.3 g) were placed in a fluid bed dryer granulator (manufactured by POWREX CORPORATION), preheated and mixed, and an aqueous solution (213.9 g) of hydroxypropylcellulose (12.8 g) was sprayed to give a granulated powder. The total amount of the obtained granulated powder was passed through a sieve No. 20 to give a sieved powder. The obtained sieved powder (360 g), sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 2

Compound A (267.4 g), D-mannitol (85.6 g), crystalline cellulose (21.4 g), and calcium silicate (12.8 g) were placed in a fluid bed dryer granulator (manufactured by POWREX CORPORATION), preheated and mixed, and an aqueous solution (213.9 g) of hydroxypropylcellulose (12.8 g) was sprayed to give a granulated powder. The total amount of the obtained granulated powder was passed through a sieve No. 20 to give a sieved powder. The obtained sieved powder (360 g), sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 3

Compound A (267.4 g), D-mannitol (94.1 g), crystalline cellulose (21.4 g), and magnesium aluminometasilicate (4.3 g) were placed in a fluid bed dryer granulator (manufactured by POWREX CORPORATION), preheated and mixed, and an aqueous solution (213.9 g) of hydroxypropylcellulose (12.8 g) was sprayed to give a granulated powder. The total amount of the obtained granulated powder was passed through a sieve No. 20 to give a sieved powder. The obtained sieved powder (360 g), sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 4

Compound A (267.4 g), D-mannitol (77 g), crystalline cellulose (21.4 g), and magnesium aluminometasilicate (21.4 g) were placed in a fluid bed dryer granulator (manufactured by POWREX CORPORATION), preheated and mixed, and an aqueous solution (209.4 g) of hydroxypropylcellulose (12.6 g) was sprayed to give a granulated powder. The total amount of the obtained granulated powder was passed through a sieve No. 20 to give a sieved powder. The obtained sieved powder (360 g), sodium starch glycolate (10.8 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Reference Example 1

Compound A (802.1 g), D-mannitol (295.2 g), and crystalline cellulose (64.2 g) were placed in a fluid bed dryer granulator (manufactured by POWREX CORPORATION), preheated and mixed, and an aqueous solution (641.7 g) of hydroxypropylcellulose (38.5 g) was sprayed to give a granulated powder. The obtained granulated powder was applied to a power mill (manufactured by SHOWA KAGAKU KIKAI CO., LTD.) to give a sieved powder.

### Reference Example 2

Compound A (802.1 g), D-mannitol (256.7 g), crystalline cellulose (64.2 g), and magnesium aluminometasilicate (38.5 g) were placed in a fluid bed dryer granulator (manufactured by POWREX CORPORATION), preheated and mixed, and an aqueous solution (641.7 g) of hydroxypropylcellulose (38.5 g) was sprayed to give a granulated powder. The obtained granulated powder was applied to a power mill (manufactured by SHOWA KAGAKU KIKAI CO., LTD.) to give a sieved powder.

### Comparative Example 3

The sieved powder (360 g) produced in Reference Example 1, sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Comparative Example 4

The sieved powder (360 g) produced in Reference Example 1, croscarmellose sodium (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Comparative Example 5

The sieved powder (360 g) produced in Reference Example 1, crospovidone (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 5

The sieved powder (360 g) produced in Reference Example 2, sodium starch glycolate (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 6

The sieved powder (360 g) produced in Reference Example 2, croscarmellose sodium (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Example 7

The sieved powder (360 g) produced in Reference Example 2, crospovidone (18 g) and magnesium stearate (5.4 g) were mixed in a polyethylene bag to give a mixed powder. The mixed powder (383.4 g) was tableted by a tableting machine (manufactured by Kikusui Seisakusho Ltd.) to give core tablets (320 mg per tablet). On the other hand, Opadry Red (86.62 g) and Opadry Yellow (173.24 g) were dissolved in purified water (2338.7 g) to give a coating agent. The obtained coating agent was sprayed on the aforementioned core tablets in a film coating machine (manufactured by Freund Corporation) to apply a coating (13 mg per tablet), whereby film-coated tablets containing 200 mg of compound A per tablet were obtained.

### Comparative Example 6

Compound A (300.0 g), D-mannitol (109.2 g), and low-substituted hydroxypropylcellulose (48.0 g) were placed in a fluid bed dryer granulator (manufactured by POWREX CORPORATION), preheated and mixed, and an aqueous solution (281.3 g) of hydroxypropylcellulose (14.4 g) and polysorbate 80 (3.6 g) was sprayed to give a granulated powder. The total amount of the obtained granulated powder was passed through a sieve No. 20 to give a sieved powder. The obtained sieved powder (396 g) and magnesium stearate (4.0 g) were mixed in a polyethylene bag to give a mixed powder. The mixed powder (400.0 g) was tableted by a tableting machine (manufactured by Kikusui Seisakusho Ltd.) to give core tablets (160 mg per tablet). On the other hand, titanium oxide (6.5 g) and red ferric oxide (0.4 g) were dispersed in purified water (100 g) and the obtained dispersion and a solution of hydroxypropyl methylcellulose 2910 (48.5 g) and macrogol 6000 (10 g) in purified water (488.6 g) were mixed to give a coating agent. The obtained coating agent was sprayed on the aforementioned core tablets in a film coating machine (manufactured by Freund Corporation) to apply a coating (6.54 mg per tablet), whereby film-coated tablets containing 100 mg of compound A per tablet were obtained.

### Comparative Example 7

Compound A (300.0 g), D-mannitol (112.8 g), and low-substituted hydroxypropylcellulose (48.0 g) were placed in a fluid bed dryer granulator (manufactured by POWREX CORPORATION), preheated and mixed, and an aqueous solution (240.0 g) of hydroxypropylcellulose (14.4 g) was sprayed to give a granulated powder. The total amount of the obtained granulated powder was passed through a sieve No. 20 to give a sieved powder. The obtained sieved powder (396 g) and magnesium stearate (4.0 g) were mixed in a polyethylene bag to give a mixed powder. The mixed powder (400.0 g) was tableted by a tableting machine (manufactured by Kikusui Seisakusho Ltd.) to give core tablets (160 mg per tablet). On the other hand, titanium oxide (6.5 g) and red ferric oxide (0.4 g) were dispersed in purified water (100 g) and the obtained dispersion and a solution of hydroxypropyl methylcellulose 2910 (48.5 g) and macrogol 6000 (10 g) in purified water (488.6 g) were mixed to give a coating agent. The obtained coating agent was sprayed on the aforementioned core tablets in a film coating machine (manufactured by Freund Corporation) to apply a coating (6.54 mg per tablet), whereby film-coated tablets containing 100 mg of compound A per tablet were obtained.

### Example 8

Compound A (300.0 g), D-mannitol (105.6 g), low-substituted hydroxypropylcellulose (48.0 g) and magnesium aluminometasilicate (3.6 g) were placed in a fluid bed dryer granulator (manufactured by POWREX CORPORATION), preheated and mixed, and an aqueous solution (281.3 g) of hydroxypropylcellulose (14.4 g) and polysorbate 80 (3.6 g) was sprayed to give a granulated powder. The total amount of the obtained granulated powder was passed through a sieve No. 20 to give a sieved powder. The obtained sieved powder (396 g) and magnesium stearate (4.0 g) were mixed in a polyethylene bag to give a mixed powder. The mixed powder (400.0 g) was tableted by a tableting machine (manufactured by Kikusui Seisakusho Ltd.) to give core tablets (160 mg per tablet). On the other hand, titanium oxide (6.5 g) and red ferric oxide (0.4 g) were dispersed in purified water (100 g) and the obtained dispersion and a solution of hydroxypropyl methylcellulose 2910 (48.5 g) and macrogol 6000 (10 g) in purified water (488.6 g) were mixed to give a coating agent. The obtained coating agent was sprayed on the aforementioned core tablets in a film coating machine (manufactured by Freund Corporation) to apply a coating (6.54 mg per tablet), whereby film-coated tablets containing 100 mg of compound A per tablet were obtained.

### Comparative Example 8

Compound A (300.0 g), D-mannitol (123.6 g), and low-substituted hydroxypropylcellulose (33.6 g) were placed in a fluid bed dryer granulator (manufactured by POWREX CORPORATION), preheated and mixed, and an aqueous solution (281.3 g) of hydroxypropylcellulose (14.4 g) and polysorbate 80 (3.6 g) was sprayed to give a granulated powder. The total amount of the obtained granulated powder was passed through a sieve No. 20 to give a sieved powder. The obtained sieved powder (396 g) and magnesium stearate (4.0 g) were mixed in a polyethylene bag to give a mixed powder. The mixed powder (400.0 g) was tableted by a tableting machine (manufactured by Kikusui Seisakusho Ltd.) to give core tablets (160 mg per tablet). On the other hand, titanium oxide (6.5 g) and red ferric oxide (0.4 g) were dispersed in purified water (100 g) and the obtained dispersion and a solution of hydroxypropyl methylcellulose 2910 (48.5 g) and macrogol 6000 (10 g) in purified water (488.6 g) were mixed to give a coating agent. The obtained coating agent was sprayed on the aforementioned core tablets in a film coating machine (manufactured by Freund Corporation) to apply a coating (6.54 mg per tablet), whereby film-coated tablets containing 100 mg of compound A per tablet were obtained.

### Example 9

Compound A (300.0 g), D-mannitol (118.8 g), low-substituted hydroxypropylcellulose (33.6 g) and magnesium aluminometasilicate (4.8 g) were placed in a fluid bed dryer granulator (manufactured by POWREX CORPORATION), preheated and mixed, and an aqueous solution (281.3 g) of hydroxypropylcellulose (14.4 g) and polysorbate 80 (3.6 g) was sprayed to give a granulated powder. The total amount of the obtained granulated powder was passed through a sieve No. 20 to give a sieved powder. The obtained sieved powder (396 g) and magnesium stearate (4.0 g) were mixed in a polyethylene bag to give a mixed powder. The mixed powder (400.0 g) was tableted by a tableting machine (manufactured by Kikusui Seisakusho Ltd.) to give core tablets (160 mg per tablet). On the other hand, titanium oxide (6.5 g) and red ferric oxide (0.4 g) were dispersed in purified water (100 g) and the obtained dispersion and a solution of hydroxypropyl methylcellulose 2910 (48.5 g) and macrogol 6000 (10 g) in purified water (488.6 g) were mixed to give a coating agent. The obtained coating agent was sprayed on the aforementioned core tablets in a film coating machine (manufactured by Freund Corporation) to apply a coating (6.54 mg per tablet), whereby film-coated tablets containing 100 mg of compound A per tablet were obtained.

### Example 10

The mixed powder obtained in Example 8 was tableted by a tableting machine to give core tablets (320 mg and 480 mg per tablet). Using the coating agent obtained in Example 8 and a film coating machine, the coating agent was sprayed on the aforementioned core tablets to apply a coating (13.08 mg per tablet and 19.62 mg per tablet), whereby film-coated tablets containing 200 mg or 300 mg of compound A per tablet were obtained.

### Example 11

The mixed powder obtained in Example 9 was tableted by a tableting machine to give core tablets (320 mg and 480 mg per tablet). Using the coating agent obtained in Example 9 and a film coating machine, the coating agent was sprayed on the aforementioned core tablets to apply a coating (13.08 mg per tablet and 19.62 mg per tablet), whereby film-coated tablets containing 200 mg or 300 mg of compound A per tablet were obtained.

### Experimental Example 1

The film-coated tablets containing magnesium aluminometasilicate and produced in Example 1 and the film-coated tablets without magnesium aluminometasilicate and produced in Comparative Example 1 were measured for the weight of core tablet and film-coated tablet, and the minimum value and maximum value thereof, as well as coefficient of variation were evaluated. In addition, the amount of compound A contained in the film-coated tablets was measured by high performance liquid chromatography, and the content of compound A in the film-coated tablet (ratio of the weight (Found) of compound A contained in film-coated tablet to charged weight of compound A in film-coated tablet) was calculated, as well as the minimum value and maximum value thereof and coefficient of variation were evaluated. As a result, as shown in [Table 1], Example 1 containing magnesium aluminometasilicate showed marked suppression of the variation of the weight of core tablet and film-coated tablet and variation of compound A content of film-coated tablet, as compared to Comparative Example 1 showing large variation.

Similarly, the film-coated tablets containing calcium silicate and produced in Example 2 were measured for the weight of the core tablet and film-coated tablet, and the minimum value and maximum value thereof, as well as coefficient of variation were evaluated. In addition, the content of compound A in the film-coated tablets was measured by high performance liquid chromatography, and the minimum value and maximum value thereof, as well as coefficient of variation were evaluated. As a result, as shown in [Table 2], various variations confirmed in Comparative Example 1 were improved in Example 2.

In each Table, n shows the number of tablets subjected to the test.

**Table 1**

| | Comparative Example 1 | Example 1 |
|---|---|---|
| average value of core tablet weight (mg) (n=20) | 323.0 | 320.1 |
| minimum value and maximum value of core tablet weight (mg) (n=20) | 311.0-336.1 | 318.3-322.0 |
| coefficient of variation (%) of core tablet weight (n=20) | 3.2 | 0.4 |
| average value of film-coated tablet weight (mg) (n=20) | 333.6 | 333.4 |
| minimum value, and maximum value of film-coated tablet weight (mg) (n=20) | 312.3-346.2 | 331.8-334.6 |
| coefficient of variation (%) of film-coated tablet weight (n=20) | 3.6 | 0.3 |
| average value of compound A content (%) of film-coated tablet (n=10) | 100.7 | 99.2 |
| minimum value and maximum value of compound A content (%) of film-coated tablet (n=10) | 93.1-108.0 | 98.5-99.6 |
| coefficient of variation (%) of compound A content of film-coated tablet (n=10) | 5.8 | 0.4 |

**Table 2**

| | Example 2 |
|---|---|
| average value of core tablet weight (mg) (n=20) | 320.3 |
| minimum value and maximum value of core tablet weight (mg) (n=20) | 316.3-324.4 |
| coefficient of variation (%) of core tablet weight (n=20) | 0.8 |
| average value of film-coated tablet weight (mg) (n=20) | 332.3 |
| minimum value, and maximum value of film-coated tablet weight (mg) (n=20) | 327.6-335.8 |
| coefficient of variation (%) of film-coated tablet weight (n=20) | 0.8 |
| average value of compound A content (%) of film-coated tablet (n=10) | 97.3 |
| minimum value and maximum value of compound A content (%) of film-coated tablet (n=10) | 96.6-99.6 |
| coefficient of variation (%) of compound A content of film-coated tablet (n=10) | 0.8 |

### Experimental Example 2

The film-coated tablet containing magnesium aluminometasilicate and produced in Example 1 and the film-coated tablet without magnesium aluminometasilicate and produced in Comparative Example 1 were stored in an opened glass bottle at 40°C/75%RH for about 3 months. The contents of dehydrated form and related substances were measured by high performance liquid chromatography and the stability of the preparations was compared. As a result, as shown in [Table 3], production of dehydrated form was remarkable and the total related substances also increased in Comparative Example 1. On the other hand, Example 1 containing magnesium aluminometasilicate showed remarkable suppression of the production of dehydrated form and increase of total related substances.

In addition, the film-coated tablet containing calcium silicate and produced in Example 2 and the film-coated tablet containing light anhydrous silicic acid and produced in Comparative Example 2 were stored in an opened glass bottle at 40°C/75%RH for about 3 months. The contents of dehydrated form and related substances were measured by high performance liquid chromatography and the stability of the preparations was compared. As a result, as shown in [Table 4], production of dehydrated form was remarkable and the total related substances also increased in Comparative Example 2. On the other hand, Example 2 containing calcium silicate did not show remarkable increase of dehydrated form or increase of total related substances.

It was confirmed that Examples 1 and 2 are stable even after preservation with time, as compared to Initial.

**Table 3**

| | Comparative Example 1 | | Example 1 | |
|---|---|---|---|---|
| | Initial | 40°C 75%RH 3 months | Initial | 40°C 75%RH 3 months |
| dehydrated form (%) | <0.02 | 0.10 | <0.02 | <0.02 |
| total related substances (%) | 0.41 | 0.57 | 0.41 | 0.43 |

**[Table 4]**

| | Comparative Example 2 | | Example 2 | |
|---|---|---|---|---|
| | Initial | 40°C 75%RH 3 months | Initial | 40°C 75%RH 3 months |
| dehydrated form (%) | 0.04 | 0.26 | <0.02 | 0.04 |
| total related substances (%) | 0.44 | 0.88 | 0.42 | 0.45 |

### Experimental Example 3

The film-coated tablets containing magnesium aluminometasilicate and produced in Examples 3 and 4 (containing 1% and 5% magnesium aluminometasilicate relative to core tablet weight) were stored in an opened glass bottle at 40°C/75%RH for about 3 months. The contents of dehydrated form and related substances were measured by high performance liquid chromatography and the stability of the preparations was compared. As a result, as shown in [Table 5], increase of dehydrated form was more remarkably suppressed in Example 4 with high magnesium aluminometasilicate content.

**[Table 5]**

| | Example 3 | | Example 4 | |
|---|---|---|---|---|
| | Initial | 40°C 75%RH 3 months | Initial 3 | 40°C 75%RH months |
| dehydrated form (%) | <0.02 | 0.04 | <0.02 | 0.02 |
| total related substances (%) | 0.40 | 0.45 | 0.40 | 0.43 |

### Experimental Example 4

The film-coated tablets produced in Example 5, Example 6 and Example 7, which contained magnesium aluminometasilicate and sodium starch glycolate, croscarmellose sodium and crospovidone, respectively, as a disintegrant, the film-coated tablets produced in Comparative Example 3, Comparative Example 4 and Comparative Example 5, which did not contain magnesium aluminometasilicate but containing sodium starch glycolate, croscarmellose sodium and crospovidone, respectively, as a disintegrant, were stored in an opened glass bottle at 40°C/75%RH for about 3 months. The contents of dehydrated form and related substances were measured by high performance liquid chromatography and the stability of the preparations was compared. As a result, as shown in [Table 6], [Table 7] and [Table 8], production of dehydrated form was remarkable and the total related substances also increased in Comparative Example group free of magnesium aluminometasilicate. On the other hand, in Examples 5 - 7 containing magnesium aluminometasilicate, production of dehydrated form and increase of the total related substances were remarkably suppressed. It was confirmed that Examples 5 - 7 are stable even after preservation with time, as compared to Initial.

**[Table 6]**

| | Comparative Example 3 | | Example 5 | |
|---|---|---|---|---|
| | Initial | 40°C 75%RH 3 months | Initial | 40°C 75%RH 3 months |
| dehydrated form(%) | <0.02 | 0.12 | <0.02 | 0.02 |
| total related substances (%) | 0.40 | 0.57 | 0.38 | 0.40 |

**[Table 7]**

| | Comparative Example 4 | | Example 6 | |
|---|---|---|---|---|
| | Initial | 40°C 75%RH 3 months | Initial | 40°C 75%RH 3 months |
| dehydrated form(%) | <0.02 | 0.18 | <0.02 | 0.04 |
| total related substances (%) | 0.38 | 0.67 | 0.38 | 0.43 |

**[Table 8]**

| | Comparative Example 5 | | Example 7 | |
|---|---|---|---|---|
| | Initial | 40°C 75%RH 3 months | Initial | 40°C 75%RH 3 months |
| dehydrated form(%) | <0.02 | 0.10 | <0.02 | 0.02 |
| total related substances (%) | 0.41 | 0.56 | 0.40 | 0.43 |

### Experimental Example 5

The film-coated tablets containing magnesium aluminometasilicate and produced in Example 8, the film-coated tablets without magnesium aluminometasilicate and produced in Comparative Example 6, and the film-coated tablets without magnesium aluminometasilicate and polysorbate 80 and produced in Comparative Example 7 were measured for the weight of core tablet and film-coated tablet, and the minimum value and maximum value thereof, as well as coefficient of variation were evaluated. In addition, the amount of compound A contained in the film-coated tablets was measured by high performance liquid chromatography, and the content of compound A in the film-coated tablet (ratio of the weight (Found) of compound A contained in film-coated tablet to charged weight of compound A in film-coated tablet) was calculated, as well as the minimum value and maximum value thereof and coefficient of variation were evaluated. As a result, as shown in [Table 9], Example 8 containing magnesium aluminometasilicate showed marked suppression of the variation of the weight of core tablet and film-coated tablet and variation of compound A content of film-coated tablet, as compared to Comparative Example 6 and Comparative Example 7 showing large variation.

Similarly, the film-coated tablets containing magnesium aluminometasilicate and produced in Example 9 and the film-coated tablets without magnesium aluminometasilicate and produced in Comparative Example 8 were measured for the weight of the core tablet and film-coated tablet, and the minimum value and maximum value thereof, as well as coefficient of variation were evaluated. In addition, the content of compound A in the film-coated tablets was measured by high performance liquid chromatography, and the minimum value and maximum value thereof, as well as coefficient of variation were evaluated. As a result, as shown in [Table 10], various variations confirmed in Comparative Example 8 were improved in Example 9.

In each Table, n shows the number of tablets subjected to the test.

**[Table 9]**

| | Comparative Example 6 | Comparative Example 7 | Example 8 |
|---|---|---|---|
| average value of core tablet weight (mg) (n=20) | 161.2 | 161.1 | 160.9 |
| minimum value and maximum value of core tablet weight (mg) (n=20) | 153.3-167.6 | 152.3-169.6 | 158.1-162.4 |
| coefficient of variation (%) of core tablet weight (n=20) | 3.1 | 3.9 | 1.0 |
| average value of film-coated tablet weight (mg) (n=20) | 167.7 | 168.9 | 166.1 |
| minimum value, and maximum value of film-coated tablet weight (mg) (n=20) | 161.6-173.8 | 155.6-179.8 | 165.1-167.0 |
| coefficient of variation (%) of film-coated tablet weight (n=20) | 2.9 | 5.3 | 0.8 |
| average value of compound A content (%) of film-coated tablet (n=10) | 99.1 | 98.4 | 98.9 |
| minimum value and maximum value of compound A content (%) of film-coated tablet (n=10) | 93.9-104.1 | 88.6-106.6 | 96.9-100.6 |
| coefficient of variation (%) of compound A content of film-coated tablet (n=10) | 3.2 | 5.9 | 0.7 |

**[Table 10]**

| | Comparative Example 8 | Example 9 |
|---|---|---|
| average value of core tablet weight (mg) (n=20) | 160.4 | 159.7 |
| minimum value and maximum value of core tablet weight (mg) (n=20) | 154.8-164.9 | 157.1-162.2 |
| coefficient of variation (%) of core tablet weight (n=20) | 2.9 | 1.1 |
| average value of film-coated tablet weight (mg) (n=20) | 166.7 | 165.9 |
| minimum value, and maximum value of film-coated tablet weight (mg) (n=20) | 160.3-171.2 | 164.3-167.1 |
| coefficient of variation (%) of film-coated tablet weight (n=20) | 2.8 | 0.9 |
| average value of compound A content (%) of film-coated tablet (n=10) | 99.4 | 98.3 |
| minimum value and maximum value of compound A content (%) of film-coated tablet (n=10) | 94.9-105.1 | 97.1-99.9 |
| coefficient of variation (%) of compound A content of film-coated tablet (n=10) | 3.3 | 0.6 |

### Experimental Example 6

The film-coated tablet containing magnesium aluminometasilicate and produced in Example 8, the film-coated tablet without magnesium aluminometasilicate and produced in Comparative Example 6, and the film-coated tablet without magnesium aluminometasilicate and polysorbate 80 and produced in Comparative Example 7 were stored in an opened glass bottle at 40°C/75%RH for about 3 months. The contents of dehydrated form and related substances were measured by high performance liquid chromatography and the stability of the preparations was compared. As a result, as shown in [Table 11], production of dehydrated form was remarkable and the total related substances also increased in Comparative Example 6 and Comparative Example 7. On the other hand, Example 8 containing magnesium aluminometasilicate showed remarkable suppression of the production of dehydrated form and increase of total related substances.

In addition, the film-coated tablet containing magnesium aluminometasilicate and produced in Example 9 and the film-coated tablet without magnesium aluminometasilicate and produced in Comparative Example 8 were stored in an opened glass bottle at 40°C/75%RH for about 3 months. The contents of dehydrated form and related substances were measured by high performance liquid chromatography and the stability of the preparations was compared. As a result, as shown in [Table 12], production of dehydrated form was remarkable, the total related substances also increased in Comparative Example 8. On the other hand, Example 9 containing magnesium aluminometasilicate showed remarkable suppression of the production of dehydrated form and increase of total related substances.

It was confirmed that Examples 8 and 9 are stable even after preservation with time, as compared to Initial.

**[Table 11]**

| | Comparative Example 6 | | Comparative Example 7 | | Example 8 | |
|---|---|---|---|---|---|---|
| | Initial | 40°C 75%RH 3 months | Initial | 40°C 75%RH 3 months | Initial | 40°C 75%RH 3 months |
| dehydrated form (%) | <0.02 | 0.09 | <0.02 | 0.09 | <0.02 | <0.02 |
| total related substances (%) | 0.41 | 0.51 | 0.41 | 0.54 | 0.41 | 0.42 |

**[Table 12]**

| | Comparative Example 8 | | Example 9 | |
|---|---|---|---|---|
| | Initial | 40°C 75%RH 3 months | Initial | 40°C 75%RH 3 months |
| dehydrated form (%) | <0.02 | 0.08 | <0.02 | <0.02 |
| total related substances (%) | 0.41 | 0.51 | 0.40 | 0.42 |

### Industrial Applicability

According to the present invention, a solid preparation containing 6-((7S)-7-hydroxy-6,7-dihydro-5H-pyrrolo[1,2-c]imidazol-7-yl)-N-methyl-2-naphthamide or a salt thereof at a high content (e.g., 40 wt% or more) as an active ingredient, which can suppress variation in preparation weight and content of active ingredient can be provided. According to the present invention, moreover, a solid preparation wherein the active ingredient is stabilized, namely, a solid preparation wherein preparation preservation stability is improved, and production or increase of dehydrated form and related substances, which are decomposed products of the active ingredient, is suppressed, can be provided.

## Claims

1. A solid preparation comprising
(1) 50 - 80 wt% of 6-((7S)-7-hydroxy-6,7-dihydro-5H-pyrrolo[1,2-c]imidazol-7-yl)-N-methyl-2-naphthamide or a salt thereof,
(2) D-mannitol and
(3) an alkaline earth metal salt selected from magnesium aluminometasilicate and calcium silicate.

2. The solid preparation according to claim 1, wherein the alkaline earth metal salt is magnesium aluminometasilicate.

3. The solid preparation according to claim 1, wherein the alkaline earth metal salt is basic magnesium aluminometasilicate.

4. The solid preparation according to claim 1, wherein the D-mannitol is produced by a spray dry production method.

5. The solid preparation according to claim 1, further comprising hydroxypropylcellulose.

6. A method of stabilizing 6-((7S)-7-hydroxy-6,7-dihydro-5H-pyrrolo[1,2-c]imidazol-7-yl)-N-methyl-2-naphthamide or a salt thereof, comprising adding (1) an alkaline earth metal salt selected from magnesium aluminometasilicate and calcium silicate and (2) D-mannitol to a solid preparation containing 6-((7S)-7-hydroxy-6,7-dihydro-5H-pyrrolo[1,2-c]imidazol-7-yl)-N-methyl-2-naphthamide or a salt thereof.

7. The method according to claim 6, wherein the alkaline earth metal salt is basic magnesium aluminometasilicate.

## Patentansprüche

1. Festes Präparat, umfassend
(1) 50 bis 80 Gew.-% 6-((7S)-7-Hydroxy-6,7-dihydro-5H-pyrrolo[1,2-c]imidazol-7-yl)-N-methyl-2-naphthamid oder ein Salz davon;
(2) D-Mannit; und
(3) ein Erdalkalimetallsalz, das aus Magnesiumaluminometasilicat und Calciumsilicat ausgewählt ist.

2. Festes Präparat gemäß Anspruch 1, wobei es sich bei dem Erdalkalimetallsalz um Magnesiumaluminometasilicat handelt.

3. Festes Präparat gemäß Anspruch 1, wobei es sich bei dem Erdalkalimetallsalz um basisches Magnesiumaluminometasilicat handelt.

4. Festes Präparat gemäß Anspruch 1, wobei das D-Mannit durch ein Sprühtrocknungsproduktionsverfahren hergestellt wird.

5. Festes Präparat gemäß Anspruch 1, das weiterhin Hydroxypropylcellulose umfasst.

6. Verfahren zum Stabilisieren von 6-((7S)-7-Hydroxy-6,7-dihydro-5H-pyrrolo[1,2-c]imidazol-7-yl)-N-methyl-2-naphthamid oder einem Salz davon, umfassend das Hinzufügen (1) eines Erdalkalimetallsalzes, das aus Magnesiumaluminometasilicat und Calciumsilicat ausgewählt ist, und (2) D-Mannit zu einem festen Präparat, das 6-((7S)-7-Hydroxy-6,7-dihydro-5H-pyrrolo[1,2-c]imidazol-7-yl)-N-methyl-2-naphthamid oder ein Salz davon enthält.

7. Verfahren gemäß Anspruch 6, wobei es sich bei dem Erdalkalimetallsalz um basisches Magnesiumaluminometasilicat handelt.

## Revendications

1. Préparation solide comprenant
(1) 50 - 80 % en poids de 6-((7S)-7-hydroxy-6,7-dihydro-5H-pyrrolo-[1,2-c]imidazol-7-yl)-N-méthyl-2-naphtamide ou d'un sel de ce composé,
(2) du D-mannitol et
(3) un sel de métal alcalino-terreux choisi parmi l'aluminométasilicate de magnésium et le silicate de calcium.

2. Préparation solide selon la revendication 1, dans laquelle le sel de métal alcalino-terreux est l'aluminométasilicate de magnésium.

3. Préparation solide selon la revendication 1, dans laquelle le sel de métal alcalino-terreux est l'aluminométasilicate de magnésium basique.

4. Préparation solide selon la revendication 1, dans laquelle le D-mannitol est produit par un procédé de production par séchage par atomisation.

5. Préparation solide selon la revendication 1, comprenant en outre de l'hydroxypropylcellulose.

6. Procédé de stabilisation de 6-((7S)-7-hydroxy-6,7-dihydro-5H-pyrrolo-[1,2-c]imidazol-7-yl)-N-méthyl-2-naphtamide ou d'un sel de ce composé, comprenant l'addition (1) d'un sel de métal alcalino-terreux choisi parmi l'aluminométasilicate de magnésium et le silicate de calcium et (2) de D-mannitol à une préparation solide contenant du 6-((7S)-7-hydroxy-6,7-dihydro-5H-pyrrolo[1,2-c]imidazol-7-yl)-N-méthyl-2-naphtamide ou un sel de ce composé.

7. Procédé selon la revendication 6, dans laquelle le sel de métal alcalino-terreux est l'aluminométasilicate de magnésium basique.
